# EUROPEAN PATENT APPLICATION

(11) **EP 4 316 751 A1**
(43) Date of publication of application: **07.02.2024**
(21) Application number: 22774348.1
(22) Date of filing: 25.03.2022
(51) Int. Cl.: B25J 15/02, B25J 17/02, B25J 9/10

(54) **MULTI-DEGREE-OF-FREEDOM INSTRUMENT FOR ROBOT**

(30) Priority: 25.03.2021 CN 202110316563
(71) Applicant: Chengdu Borns Medical Robotics Inc., Chengdu, Sichuan 610041 (CN)
(72) Inventor: LI, Yao, Chengdu, Sichuan 610041 (CN); LI, Zhiqiang, Chengdu, Sichuan 610041 (CN)
(74) Representative: Bryn Aarflot AS
(86) International application number: PCT/CN2022/083078
(87) International publication number: WO 2022/199695

(57) **Abstract**

The invention provides a multi-degree-of-freedom instrument for robot, which comprises an instrument base, an instrument rod and an instrument pliers head, wherein one end of the instrument rod is rotatably arranged on the instrument base, the instrument rod can rotate around a central axis of the instrument rod, the instrument pliers head is hinged to the other end of the equipment rod, and the instrument base can drive the instrument pliers heads and the equipment rod to act. The invention has the advantages of multiple degrees of freedom, high flexibility and high reliability.

## Description

The present invention claims priority from the Chinese application NO CN202110316563.1, the patent was submitted to the Chinese Patent Office on March 25, 2021, the patent application with the title of "Multi-degree-of-freedom instrument for robot", Which is incorporated herein by reference in its entirety.

### Technical field

The invention relates to the technical field of robot manipulators, in particular to a multi-degree-of-freedom instrument for robot.

### Background

With the continuous improvement of artificial intelligence technology, robots have been increasingly widely used in daily life, production and surgery, and have became the development direction of intelligent robots. At the same time, due to the limitation of the operating space of the robot in a specific working environment, there are higher requirements for the reliability and flexibility of the robot manipulator. Therefore, how to design a multi-Dof robot manipulator with multi-Dof, high flexibility and high reliability, which can adapt to the robot and assist the operator to complete precise and complex operations, has become one of the technical problems to be solved in the development of intelligent robots.

### Summary of the Invention

In view of the above problems in the prior part, the current invention proposes a multi-degree-of-freedom instrument for robot, which has multiple degrees of freedom, high flexibility, and high reliability.

In the first perspective, the present invention provides a multi-degree-of-freedom instrument for robot, comprising an instrument base, an instrument rod and an instrument forceps head, one end of the instrument rod is rotatably disposed on the instrument base, the instrument rod is capable of rotating around its central axis, and the instrument forceps head is hinged to the other end of the instrument rod; the instrument base can drive the instrument forceps head and the instrument rod to act.

In one embodiment, the instrument forceps head comprises a finger joint and a wrist joint, the finger joint is used to perform an opening and closing movement and/or rotation, one end of the wrist joint hinged with the finger joint, and the other end of wrist joint hinged on the instrument rod, wherein the plane of motion of the finger joint and the plane of motion of the wrist joint are perpendicular to each other.

The beneficial effect of the embodiment is that the instrument forceps head can realize movement with two degrees of freedom.

In one embodiment, the finger joint comprises a first jaw, a second jaw, a first rope guide wheel, a second rope guide wheel and a finger joint wheel shaft, the clamping end of the second jaw can be meshed with the clamping end of the first jaw; And the first rope guide wheel is integrally arranged at the end of the first jaw away from the clamping end of the first jaws, which is used to drive the first jaw to act, and the second rope guide wheel is integrally arranged at the end of the second jaw away from the clamping end of the second jaw, which is used to drive the second jaw to act; the first rope guide wheel and the second rope guide wheel are coaxially arranged, and the finger joint wheel shaft penetrates through the central shafts of the first rope guide wheel and the second rope guide wheel and is fixed on the wrist joint; and the first rope guide wheel and the second rope guide wheel can rotate relative to each other to drive the first jaw and the second jaw to rotate relative to each other, to realize the opening and closing movement of the finger joint; the first rope guide wheel and the second rope guide wheel synchronously rotate to drive the first jaw and the second jaw to synchronously rotate so as to realize the rotation of the finger joint.

The embodiment has the beneficial effects that the opening and closing motion of the finger joint is realized through the relative rotation motion of the first jaw and the second jaw, the rotation of the finger joint is realized through the synchronous rotation movement of the first jaw and the second jaw, and the hinging between the finger joint and a wrist joint is realized by rotating the first rope guide wheel and the second rope guide wheel around a finger joint wheel shaft.

In one embodiment, the wrist joint comprises a wrist joint body, a third rope guide wheel and a wrist joint wheel shaft, wherein the finger joint wheel shaft is arranged at one end of the wrist joint body. The third rope guide wheel is integrally arranged at one end of the wrist joint body far away from the finger joint wheel shaft and is used for driving the wrist joint body to act. The wrist joint wheel shaft penetrates through the central shaft of the third rope guide wheel and is fixed on the instrument rod, and the third rope guide wheel can rotate around the wrist joint wheel shaft to realize the wrist joint hinged to the instrument rod.

The embodiment has the beneficial effect that the third rope guide wheel rotates around the wrist joint wheel shaft to realize the wrist joint hinged to the instrument rod.

In one embodiment, the instrument base comprises an instrument base housing, a rotating module, a first finger joint module, a second finger joint module and a wrist joint module, wherein the main body of the rotating module is a rotating shaft which is rotatably arranged on the instrument base housing and can rotate around the central axis of the rotating shaft; One end of the instrument rod far away from the wrist joint is rotatably arranged on the instrument base housing, the rotating shaft is parallel to the instrument rod, one end of a first twist rope is clockwise wound on the upper part of the rotating shaft, the other end of the first twist rope is anticlockwise wound on the instrument rod corresponding to the upper part of the rotating shaft; One end of a second twist rope is anticlockwise wound at the lower part of the rotating shaft, the other end of the second twist rope is wound on the instrument rod corresponding to the lower part of the rotating shaft clockwise, the rotating shaft can drive the instrument rod to rotate in the rotating process; the main body of the first finger joint module is a first finger joint rotating shaft which is rotatably arranged on the instrument base housing and can rotate around the central axis, the third twist rope is in power transmission with the first rope guide wheel, one end of the third twist rope is wound on the upper part of the first finger joint rotating shaft clockwise, the other end of the third twist rope is wound on the lower part of the first finger joint rotating shaft anticlockwise, the first finger joint rotating shaft can drive the first rope guide wheel to rotate in the rotating process, which is used to drive the first jaw to move, the main body of that second finger joint module is a second finger joint rotating shaft, the second finger joint rotating shaft is rotatively arranged on a the instrument base housing, the second finger joint rotating shaft can rotate around a central axis of the second finger joint rotating shaft, a fourth twist rope is in power transmission with the second rope guide wheel, one end of the fourth twist rope is wound on the upper part of the second finger joint rotating shaft clockwise, the other end is counterclockwise wound on the lower part of the second finger joint rotating shaft, the second finger joint rotating shaft can drive the second rope guide wheel to rotate in the rotating process, which is used to drive the second jaw to move, the main body of the wrist joint module is a wrist joint rotating shaft which is rotatively arranged on the instrument base housing and can rotate around the central axis thereof, the fifth twist rope is in power transmission with the third rope guide wheel; one end of the fifth twist rope is wound on the upper part of the wrist joint rotating shaft clockwise, and the other end of the fifth twist rope is wound on the lower part of the wrist joint rotating shaft anticlockwise, the wrist joint rotating shaft can drive the third rope guide wheel to rotate in the rotation process, which is used to drive the wrist joint to move.

The embodiment has the beneficial effects that the first twist rope and the second twist rope are wound, so that the rotating shaft can drive the instrument rod to rotate clockwise or counterclockwise in the process of rotating clockwise or counterclockwise. The third twist rope and the first rope guide wheel adopt rope and wheel transmission, which converts the rotating motion of the first finger joint rotating shaft into the rotating motion of the first rope guide wheel, so as to drive the first jaw to act. The fourth twist rope and the second rope guide wheel adopt rope and wheel transmission, which converts the rotating motion of the second finger joint rotating shaft into the rotating motion of the second rope guide wheel, so as to drive the second jaw to act, and the first jaw and the second jaw realize object clamping in the process of being close to each other. The fifth twist rope and the third rope guide wheel adopt rope and wheel transmission, which converts the rotating motion of the wrist joint rotating shaft into the rotating motion of the third rope guide wheel, so as to drive the wrist joint body to act and further realize the wrist joint action.

In one embodiment, the instrument base further comprises a mounting buckle which is arranged on the instrument base housing and is used for realizing the clamping connection between the multi-degree-of-freedom instrument for robot and the robot.

The beneficial effect of the embodiment is that the clamping connection between the multi-degree-of-freedom instrument for robot and the robot is realized through the arrangement of the mounting buckle.

In one embodiment, the instrument rod is a hollow shaft, and the third twist rope, the fourth twist rope, and the fifth twist rope respectively pass through the instrument rod.

The embodiment has the advantage of protecting the twist rope.

In one embodiment, the wrist joint is provided with a pulley block, and the third twist rope and the fourth twist rope respectively bypass the pulley block for reversing and tensioning the third twist rope and the fourth twist rope.

The embodiment has the advantage of realizing the reversing and tensioning of the third twist rope and the fourth twist rope.

In one embodiment, at least three first pulleys are arranged on the instrument base housing, the third twist rope, the fourth twist rope and the fifth twist rope respectively go around the first pulleys corresponding to the third twist rope, the fourth twist rope and the fifth twist rope to realize the direction change of the third twist rope, the forth twist rope and fifth twist rope.

The embodiment has the beneficial effect that the third twist rope, the fourth twist rope and the fifth twist rope are used for reversing.

Compared with the prior art, the invention has the advantages that:
(1) Multiple degrees of freedom, high flexibility and high reliability.
(2) That structure is simple and the operation is convenient.
(3) Realizing the actions of the first jaw, the second jaw and the wrist joint by adopting a rope and wheel power transmission mode.
(4) The first twist rope and the second twist rope are wound in such a way that the rotating shaft can drive the instrument rod to rotate clockwise or counterclockwise in the process of clockwise or counterclockwise rotation.
(5) The third twist rope, the fourth twist rope, and the fifth twist rope are wound in such a manner as to convert the rotational motion of the first finger joint rotating shaft into the rotational motion of the first rope guide wheel, convert the rotational movement of the second finger joint rotating shaft into the rotational movement of the second rope guide wheel, and convert the rotation of the wrist joint rotating shaft to the rotation of the third rope guide wheel respectively.

The features described above may be combined in any suitable manner or replaced by equivalent features so long as the object of the invention is achieved.

### Brief Description of the Drawings

Hereinafter, the present invention will be described in more detail on the basis of embodiments and with reference to the accompanying drawings. Among
Fig. 1 shows a first isometric view of an end effector of multi-degree-of-freedom instrument for robot;
Fig. 2 shows an isometric view of an instrument base of multi-degree-of-freedom instrument for robot;
Fig. 3 shows a second isometric view of an end effector of multi-degree-of-freedom instrument for robot;
Fig. 4 shows the winding structure of the third twist rope at the first angle;
Fig. 5 shows the winding structure of the third twist rope at the second angle;
Fig. 6 show a side view of that end effector;
Fig. 7 show a side view of that wrist joint removed by end effector;
Fig. 8 show an isometric view of that wrist joint removed by end effector;
Fig. 9 shows a structure diagram of an instrument base;
Fig. 10 shows a front view of the instrument base;
Fig. 11 shows a bottom view of the instrument base;
Fig. 12 shows a top view of the instrument base;
Fig. 13 shows a right side view of the instrument base;
Fig. 14 shows a left side view of the instrument base;

In the drawings, like parts are given like reference numerals. The drawings are not to scale.

10- multi-degree-of-freedom instrument for robot; 11- instrument base; 111-instrument base housing; 113- rotating module; 113a- rotating shaft; 113b- first twist rope; 113c- second twist rope; 113d- first knob; 115- first finger joint module; 115a- first finger joint rotating shaft; 115b- third twist rope; 115c- second knob; 117- second finger joint module; 117a-second finger joint rotating shaft; 117b- fourth twist rope; 117c- third knob; 119- wrist joint module; 119a- wrist joint rotating shaft; 119b- fifth twist rope; 119c- fourth knob; 123-mounting buckle; 125- unlocking button; 127- identification module; 13- instrument rod; 15-instrument forceps head; 151- finger joint; 151a- first jaw; 151b-second jaw; 151c- first rope guide wheel; 151d- second rope guide wheel; 151e- finger joint wheel shaft; 153- wrist joint; 153a- wrist joint body; 153b- third rope guide wheel; 153c- wrist Joint wheel shaft; 155- pulley block; 155a- second pulley; 155b- third pulley.

### Detailed Description of the Invention

In order that the present invention may be more fully understood, reference will now be made to the accompanying drawings, in which:. Preferred embodiments of the present invention are illustrated in the accompanying drawings. This invention may, however, be embodied in many different forms and is not limited to the embodiments described herein.

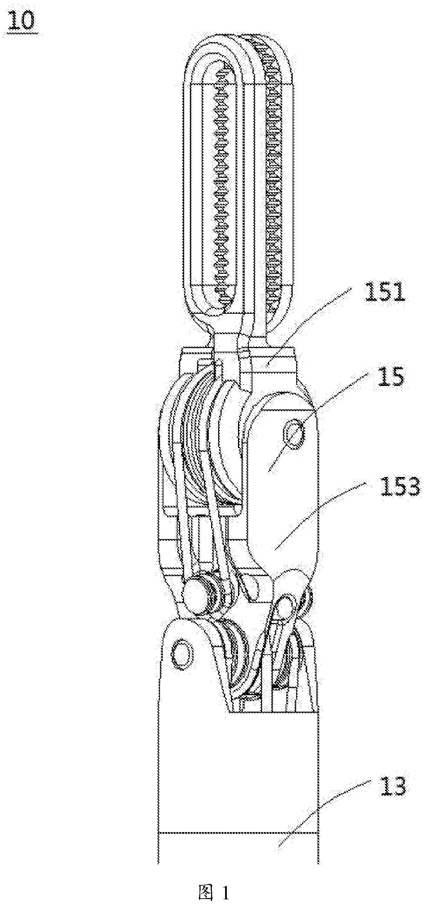

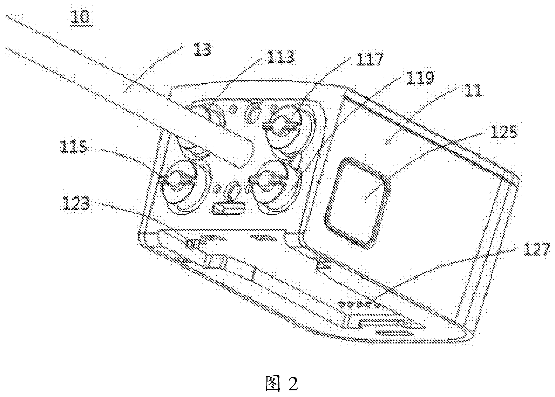

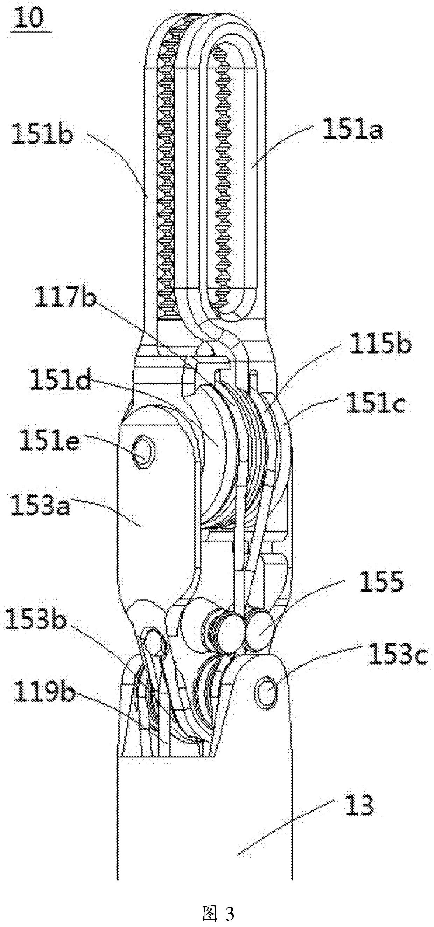

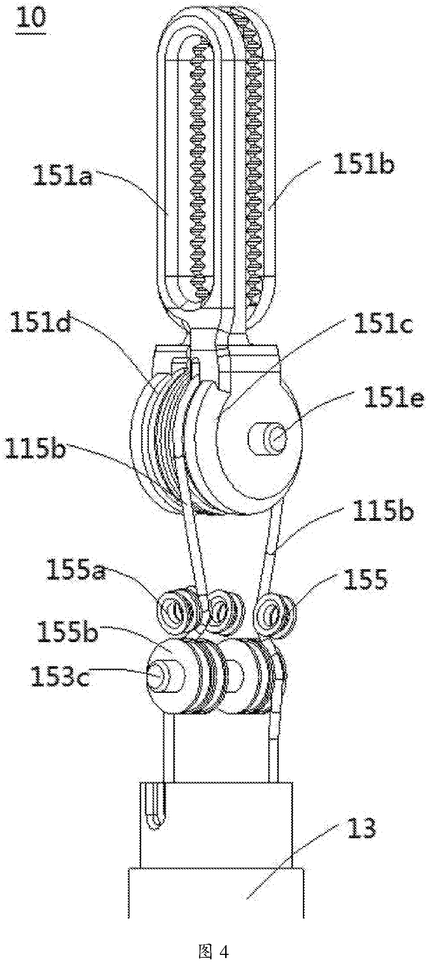

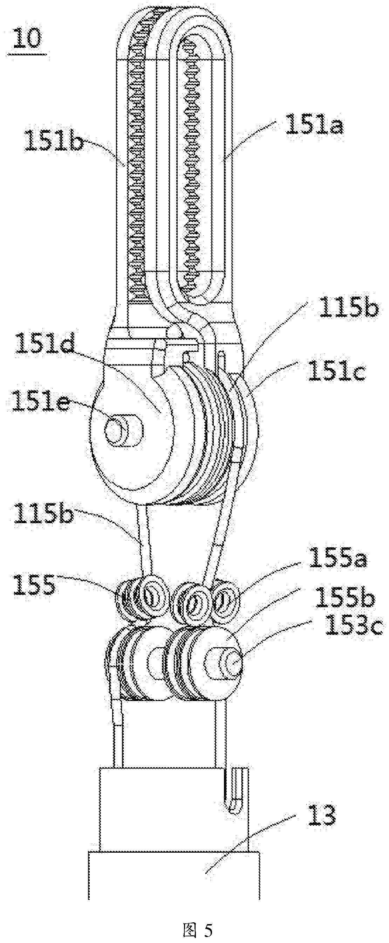

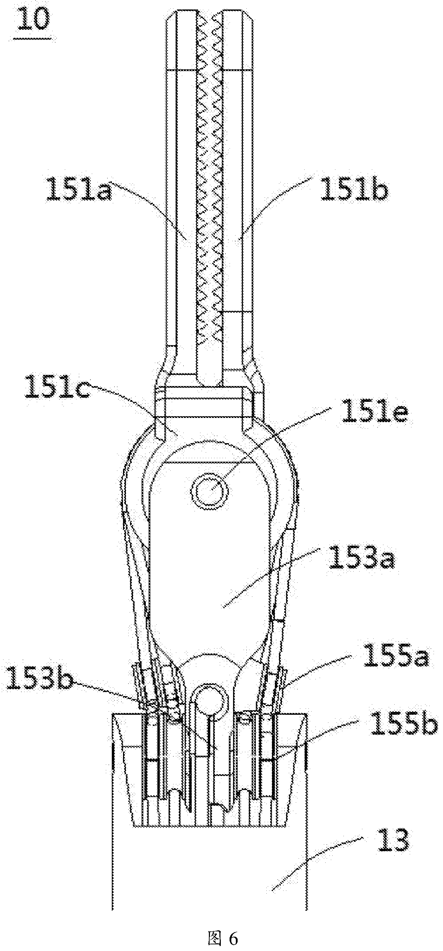

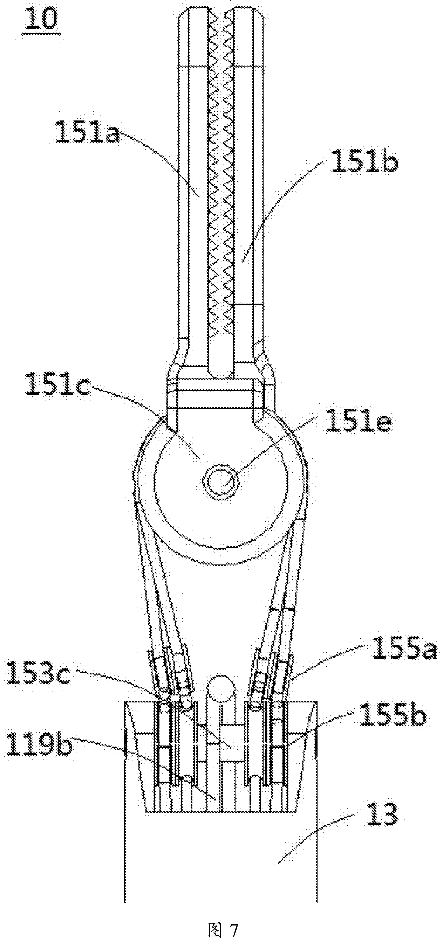

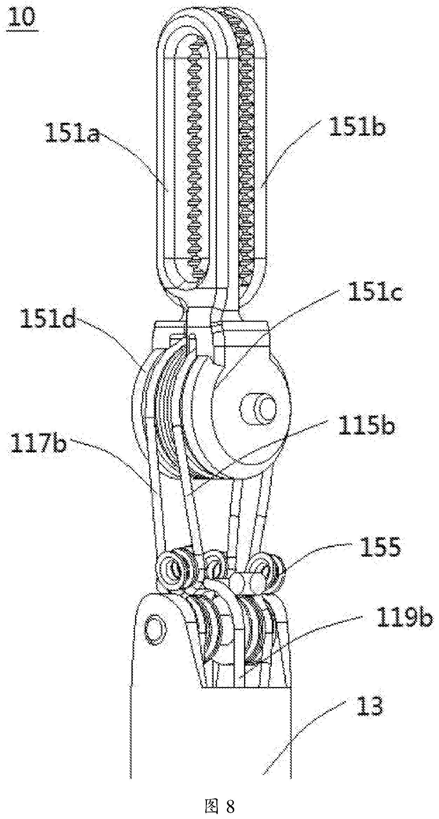

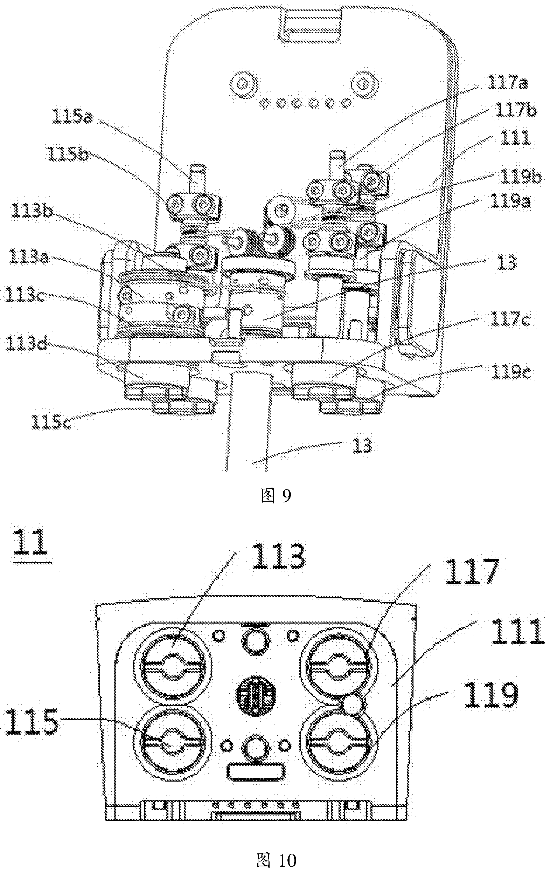

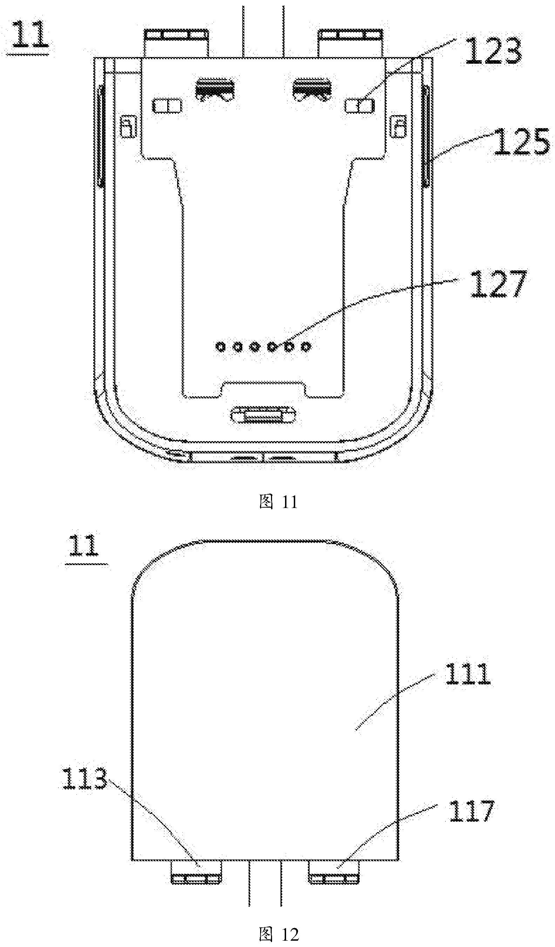

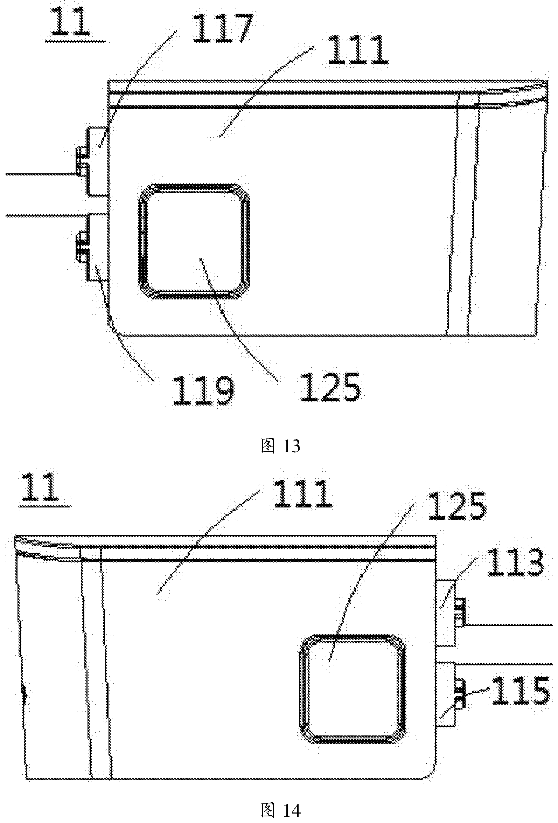

As shown in figs. 1-14, a multi-degree-of-freedom instrument for robot 10 comprises an instrument base 11, an instrument rod 13 and an instrument forceps head 15, wherein one end of the instrument rod 13 is rotatably disposed on the instrument base 11, the instrument rod 13 is capable of rotating around a central axis, the instrument forceps head 15 is hinged to the other end of the instrument rod 13; the instrument base 11 can drive the instrument forceps head 15 and the instrument rod 13 to move.

Among them, the instrument forceps head 15 comprises a finger joint 151 and a wrist joint 153, the finger joint 151 is used to perform an opening and closing movement and/or rotation, one end of the wrist joint 153 hinged with the finger joint 151, and the other end of wrist joint 153 hinged on the instrument rod 13, the plane of motion of the finger joint 151 and the plane of motion of the wrist joint 153 are perpendicular to each other.

The finger joint 151 includes a first jaw 151a, a second jaw 151b, a first rope guide wheel 151c, a second rope guide wheel 151d and a finger joint wheel shaft 151e, the clamping end of the second jaw 151b can be meshed with the clamping end of the first jaw 151a, the first rope guide wheel 151c is integrally arranged at the end of the first jaw 151a far away from the clamping end of the first jaw 151a, and is used for driving the first jaw 151a to act; the second rope guide wheel 151d is integrally arranged at the end of the second jaw 151b far away from the clamping end of a second jaw, and is used for driving the second jaws 151b to act; the first rope guide wheel 151c and the second rope guide wheel 151d are coaxially arranged, the finger joint wheel shaft 151e penetrates through the central shafts of the first rope guide wheel 151c and the second rope guide wheel 151d and is fixed on the wrist joint 153, and the first rope guide wheel 151c and the second rope guide wheel 151d can rotate around the finger joint wheel shaft 151e for realizing the hinging of the finger joint 151 and the wrist joint 153. The first rope guide wheel 151c and the second rope guide wheel 151d can rotate relative to each other to drive the first jaw 151a and the second jaw 151b to rotate relative to each other, so as to realize the opening and closing movement of the finger joint 151. The first rope guide wheel 151c and the second rope guide wheel 151d synchronously rotate to drive the first jaw 151a and the second jaw 151b to synchronously rotate, so as to realize the deflection movement of the finger joint 151.

Specifically, in this embodiment, both the clamping end of the first jaw 151a and the clamping end of the second jaw 151b are provided with clamping teeth, and the clamping teeth on the first jaw 151a and those on the second jaws 151b are distributed in a staggered manner and can be meshed with each other.

The wrist joint 153 comprises a wrist joint body 153a, a third rope guide wheel 153b and a wrist joint wheel shaft 153c, one end of the wrist joint body 153a is provided with a finger joint wheel shaft 151e, the third rope guide wheel 153b is integrally provided at one end of the wrist joint body 153 away from the finger joint wheel shaft 151e and is used for driving the wrist joint body 153a to act, the wrist joint wheel shaft 153c penetrates through a central shaft of the third rope guide wheel 153b and is fixed on the instrument rod 13, and the third rope guide wheel 153b can rotate around the wrist joint wheel shaft 153c to realize the wrist joint 153 hinged to the instrument rod 13.

Specifically, in this embodiment, the finger joint wheel shaft 151e and the wrist joint wheel shaft 153c are perpendicular to each other, the first jaw 151a and the second jaw 151b can rotate around the finger joint wheel shaft 151e, and the wrist joint body 153a can rotate around the wrist joint wheel shaft 153c, so that the plane of motion of the first jaw 151a and the second jaw 151b is perpendicular to the plane of motion of the wrist joint 153.

In one embodiment, the finger joint wheel shaft 151e is fixedly or movably connected to the wrist joint body 153a, and the wrist joint wheel shaft 153c is fixedly or movably connected to the end of the instrument rod 13.

The instrument base 11 includes an instrument base housing 111, a rotating module 113, a first finger joint module 115, a second finger joint module 117, and a wrist joint module 119.

The main body of the rotating module 113 is a rotating shaft 113a, the rotating shaft 113a is rotatably arranged on the instrument base housing 111, the rotating shaft 113a can rotate around the central axis thereof, one end of the instrument rod 13 far away from the wrist joint 153 is rotatably arranged on the instrument base housing 111, and the rotating shaft 113a is parallel to the instrument rod 13. One end of the first twist rope 113b is wound clockwise around an upper portion of the rotating shaft 113a and the other end thereof is wound counterclockwise around the instrument rod 13 corresponding to an upper portion of the rotating shaft 113a, one end of the second twist rope 113c is wound counterclockwise around a lower part of the rotating shaft 113a, the other end is wound clockwise on the instrument rod 13 corresponding to the lower part of the rotating shaft 113a, and the rotating shaft 113a can drive the instrument rod 13 to rotate during the rotation process, thereby driving the instrument forceps head 15 to rotate.

The main body of the first finger joint module 115 is a first finger joint rotating shaft 115a, the first finger joint rotating shaft 115a is rotatably arranged on the instrument base housing 111, the first finger joint rotating shaft 115a can rotate around the central axis thereof, the third twist rope 115b is in power transmission connection with the first rope guide wheel 151c, One end of the third twist rope 115b is wound on the upper portion of the first finger joint rotating shaft 115a clockwise, and the other end is wound on the lower portion of the first finger joint rotating shaft 115a counterclockwise. The first finger joint rotating shaft 115a can drive the first rope guide wheel 151c to rotate in the rotating process, which is used to drive the first jaw 151a to move.

The main body of the second finger joint module 117 is a second finger joint rotating shaft 117a, the second finger joint rotating shaft 117a is rotated on the instrument base housing 111, the second finger Joint rotating shaft 117b can rotate around the central axis thereof, the fourth twist rope 117b is in power transmission connection with the second rope guide wheel 151d, One end of the fourth twist rope 117b is wound on the upper portion of the second finger joint rotating shaft 117a clockwise, and the other end of the fourth twist rope 117b is wound on the lower portion of the second finger joint rotating shaft 117a counterclockwise. The second rope guide wheel 151d can be driven to rotate during the rotation of the second finger joint rotating shaft 117a, so as to drive the second jaw 151b to move.

The main body of the wrist joint module 119 is a wrist joint rotating shaft 119a, the wrist joint rotating shaft 119a is rotatably arranged on the instrument base housing 111, the wrist joint rotating shaft 119a can rotate around its central axis, and the fifth twist rope 119b is in power transmission connection with the third rope guide wheel 153b. One end of the fifth twist rope 119b is wound clockwise around the upper portion of the wrist joint rotating shaft 119a, and the other end of the fifth twist rope 119b is wound counterclockwise around the lower portion of the wrist joint rotating shaft 119a, so that the third rope guide wheel 153b can be driven to rotate during the rotation of the wrist joint rotating shaft 119 to drive the wrist joint 153 to move.

Specifically, in this embodiment, the rotating module 113 further includes a first knob 113d, the first knob 113d is connected to the rotating shaft 113a through a coupling, and the rotating shaft 113a can be driven to rotate by rotating the first knob 113d, thereby driving the instrument rod 13 to rotate.

The first finger joint module 115 further includes a second knob 115c. The second knob 115c is connected to the first finger joint rotating shaft 115a through a coupling, and the first finger joint rotating shaft 115a can be driven to rotate by rotating the second knob 115, so as to drive the first jaw 151a to act. The third twist rope 115b passes around the first rope guide wheel 151c and is in power transmission connection with the first rope guide wheel 151c. That is, the third twist rope 115b passes around the first rope guide wheel 151c and is in contact with them (similar to the connection between a belt and a pulley), and the first rope guide wheel 151c is able to rotate under the drive of the third twist rope 115b. One end of the third twist rope 115b is wound clockwise around an upper portion of the first finger joint rotating shaft 115a, and the other end of the third twist rope 115b is wound counterclockwise around a lower portion of the first finger joint rotating shaft 115a, so that the first finger Joint rotating shaft 115a can drive the first rope guide wheel 151c to rotate clockwise or counterclockwise during clockwise or counterclockwise rotation. The rotating direction of the first rope guide wheel 151c is the same as the rotating direction of the first finger joint rotating shaft 115a.

The second finger joint module 117 further includes a third knob 117c, the third knob 117c is connected to the second finger joint rotating shaft 117a through a coupling, and the second finger joint rotating shaft 117a can be driven to rotate by rotating the third knob 117, so as to drive the second jaw 151b to act. The fourth twist rope 117b rounds the second rope guide wheel 151d and is in power transmission connection with the second rope guide wheel 151d, that is, the fourth twist rope 117b rounds the second rope guide wheel 151d and is in contact with the second rope guide wheel 151d, and the second rope guide wheel 151d is able to rotate under the drive of the fourth twist rope 117b. One end of the fourth twist rope 117b is wound on an upper portion of the second finger joint rotating shaft 117a in a clockwise direction, and the other end of the fourth twist rope 117b is wound on a lower portion of the second finger joint rotating shaft 117a in an anticlockwise direction, so that the second finger joint rotating shaft 117a can drive the second rope guide wheels 151d to rotate in a clockwise direction or in an anticlockwise direction. The rotating direction of the second rope guide wheel 151d is the same as the rotating direction of the second finger joint rotating shaft 117a.

The wrist joint module 119 further includes a fourth knob 119c, the fourth knob 119c is connected to the wrist joint rotating shaft 119a through a coupling, and the wrist joint rotating shaft 119a can be driven to rotate by rotating the fourth knob 119c, so as to drive the wrist joint body 153a to act, and further drive the wrist joint 153 to act. A fifth twist rope 119b rounds that third rope guide wheel 153b and is in pow transmission connection with the third rope guide wheel 153b, namely, the fifth twist rope 119b rounds the third rope guide wheel 153b and is in contact with the third rope guide wheel 153b, and the third rope guide wheel 153b is able to rotate under the drive of the fifth twist rope 119b.One end of the fifth twist rope 119b is wound on the upper portion of the wrist joint rotating shaft 119a clockwise, and the other end of the fifth twist rope 119b is wound on the lower portion of the wrist joint rotating shaft 119a anticlockwise, so that the wrist joint rotating shaft 119a can drive the third rope guide wheel 153b to rotate in a clockwise direction or in an anticlockwise direction, the rotating direction of the third rope guide wheel 153b is the same as the rotating direction of the wrist joint rotating shaft 119a.

In one embodiment, because the first jaw 151a, the second jaw 151b, and the wrist joint body 153a have a relatively small amount of motion, in order to avoid loosening of the twist rope, the third twist rope 115b, the fourth twist rope 117b, and the fifth twist rope 119b may be fixedly connected to a contact portion of the first rope guide wheel 151c, the second rope guide wheel 151d, and the third rope guide wheel 153b, respectively.

In one embodiment, the rotating shaft 113a, the first finger joint rotating shaft 115a, the second finger joint rotating shaft 117a, the wrist joint rotating shaft 119a, and the instrument rod 13 are parallel to one another.

The instrument rod 13 is a hollow rod, and the third twist rope 115b, the fourth twist rope 117b, and the fifth twist rope 119b respectively pass through the instrument rod.

The wrist joint 153 is provided with a pulley block 155, and the third twist rope 115b and the fourth twist rope 117b respectively go around the pulley block 155 for reversing and tensioning the third twist rope 115b and the fourth twist rope 117b.

At least three first pulleys are provided on the instrument base housing 111, and the third twist rope 115b, the fourth twist rope 117b, and the fifth twist rope 119b are respectively wound around the first pulleys corresponding to the third twist rope 115b, the fourth twist rope 117b, and the fifth twist rope 119b, so as to realize the direction change of the third threaded rope 115b and the fourth threaded rope 117b.

Specifically, in this embodiment, the wrist joint 153 is provided with two sets of pulley blocks 155, the two sets of pulley blocks 155 are respectively provided on both sides of the wrist joint body 153 a, and each set of pulley block 155 comprises at least one second pulley 155a and at least one third pulley 155b. The moving plane of the second pulley 155a and the third pulley 155b is the same as the moving plane of the wrist joint rotating shaft 119a, and the third twist rope 115b and the fourth twist rope 117b positioned on the same side of the wrist joint body 153a respectively pass through the second pulley 155a and the third pulley 155b, Thus, the reversing and tensioning of the third twist rope 115b and the fourth twist rope 117b are achieved, the strand is prevented from being loosened during movement, and the twist rope is allowed to follow a predetermined path.

In one embodiment, the two groups of pulley blocks 155 are respectively arranged on the side surface where the end of the wrist joint rotating shaft 119a is located and on the wrist joint body 153a, so that the motion plane of the two second pulleys 155a is the same as the motion plane of the wrist joint rotating shaft 119a. It is ensured that the third twist rope 115b and the fourth twist rope 117b do not pull the wrist joint 153 to be unable to move.

The reversed third twist rope 115b, fourth twist rope 117b, and fifth twist rope 119b pass through the hollow hole of the instrument rod 13, respectively, and the instrument rod 13 protects the third twist rope 115b, fourth twist rope 117b, and fifth twist rope 119b. The three first pulleys are respectively arranged at the outlet end of the instrument rod 13 close to the instrument base housing 111. The third twist rope 115b, the fourth twist rope 117b and the fifth twist rope 119b passing through the instrument rod 13 are respectively wound around the first finger joint rotating shaft 115a, the second finger joint rotating shaft 117a and the wrist joint rotating shaft 119a after passing through the corresponding first pulleys.

The instrument base 11 further comprises a mounting buckle 123, which is arranged on the instrument base housing 111 and is used for realizing the clamping connection between the multi-degree-of-freedom instrument for robot 10 and the robot.

Specifically, in this embodiment, the instrument base 11 is further provided with an unlocking button 125 for controlling the action of the mounting buckle 123, and the action of the mounting buckle 123 can be controlled by pressing the unlocking button 125, so as to realize the assembly and disassembly of the multi-degree-of-freedom instrument for robot 10 and the robot.

In one embodiment, the first twist rope 113b, the second twist rope 113c, the third twist rope 115b, the fourth twist rope 117b, and the fifth twist rope 119b may all be wire ropes.

In one embodiment, the instrument base 11 is further provided with an identification module 127, and the identification module 127 can identify the type and identity of the robot clamped with the multi-degree-of-freedom instrument for robot 10.

In the description of the present invention, it should be understood that the orientations or positional relationships indicated by the terms "upper," lower, "bottom," top, "front," rear, "inner, outer, left, right, and the like are based on the orientations or positional relationships shown in the drawings only for convenience of description of the present invention and simplification of description, It is not intended to indicate or imply that the devices or elements referred to must have a particular orientation, be constructed and operate in a particular orientation, and therefore, should not be construed as limiting the invention.

Although the present invention has been described herein with reference to particular embodiments, it is to be understood that these embodiments are merely illustrative of the principles and applications of the present invention. It is therefore to be understood that many modifications may be made to the exemplary embodiments and that other arrangements may be devised without departing from the spirit and scope of the invention as defined by the appended claims. It is to be understood that different dependent claims and features described herein may be combined in ways other than those described in the original claims. It will also be appreciated that features described in connection with a single embodiment may be used in other described embodiments.

## Claims

1. A multi-degree-of-freedom instrument for robot, comprising an instrument base, an instrument rod and an instrument forceps head, one end of the instrument rod is rotatably disposed on the instrument base, the instrument rod is capable of rotating around its central axis, and the instrument forceps head is hinged to the other end of the instrument rod; the instrument base can drive the instrument forceps head and the instrument rod to act; the instrument forceps head comprises a finger joint and a wrist joint, the finger joint is used to perform an opening and closing movement and/or rotation, one end of the wrist joint hinged with the finger joint, and the other end of wrist joint hinged on the instrument rod, wherein the plane of motion of the finger joint and the plane of motion of the wrist joint are perpendicular to each other.

2. The multi-degree-of-freedom instrument for robot according to claim 1, wherein the finger joint comprises a first jaw, a second jaw, a first rope guide wheel, a second rope guide wheel and a finger joint wheel shaft, the clamping end of the second jaw can be meshed with the clamping end of the first jaw; and the first rope guide wheel is arranged at the end of the first jaw away from the clamping end of the first jaws, which is used to drive the first jaw to act, and the second rope guide wheel is arranged at the end of the second jaw away from the clamping end of the second jaw, which is used to drive the second jaw to act; the first rope guide wheel and the second rope guide wheel are coaxially arranged, and the finger joint wheel shaft penetrates through the central shafts of the first rope guide wheel and the second rope guide wheel and is fixed on the wrist joint; the first rope guide wheel and the second rope guide wheel can rotate around the finger joint wheel shaft for realizing the hinging of the finger joint and the wrist joint; and the first rope guide wheel and the second rope guide wheel can rotate relative to each other to drive the first jaw and the second jaw to rotate relative to each other, to realize the opening and closing movement of the finger joint; the first rope guide wheel and the second rope guide wheel synchronously rotate to drive the first jaw and the second jaw to synchronously rotate so as to realize the rotation of the finger joint.

3. The multi-degree-of-freedom instrument for robot according to claim 2, wherein the wrist joint comprises a wrist joint body, a third rope guide wheel and a wrist joint wheel shaft, wherein the finger joint wheel shaft is arranged at one end of the wrist joint body, the third rope guide wheel is arranged at one end of the wrist joint body far away from the finger joint wheel shaft and is used for driving the wrist joint body to act, the wrist joint wheel shaft penetrates through the central shaft of the third rope guide wheel and is fixed on the instrument rod, and the third rope guide wheel can rotate around the wrist joint wheel shaft to realize the wrist joint hinged to the instrument rod.

4. The multi-degree-of-freedom instrument for robot according to claim 3, wherein the instrument base comprises an instrument base housing, a rotating module, a first finger joint module, a second finger joint module and a wrist joint module, wherein the main body of the rotating module is a rotating shaft which is rotatably arranged on the instrument base housing and can rotate around the central axis of the rotating shaft; One end of the instrument rod far away from the wrist joint is rotatably arranged on the instrument base housing, the rotating shaft is parallel to the instrument rod, one end of a first twist rope is clockwise wound on the upper part of the rotating shaft, the other end of the first twist rope is anticlockwise wound on the instrument rod corresponding to the upper part of the rotating shaft; One end of a second twist rope is anticlockwise wound at the lower part of the rotating shaft, the other end of the second twist rope is wound on the instrument rod corresponding to the lower part of the rotating shaft clockwise, the rotating shaft can drive the instrument rod to rotate in the rotating process; the main body of the first finger joint module is a first finger joint rotating shaft which is rotatably arranged on the instrument base housing and can rotate around the central axis, the third twist rope is in power transmission with the first rope guide wheel, one end of the third twist rope is wound on the upper part of the first finger joint rotating shaft clockwise, the other end of the third twist rope is wound on the lower part of the first finger joint rotating shaft anticlockwise, the first finger joint rotating shaft can drive the first rope guide wheel to rotate in the rotating process, which is used to drive the first jaw to move; the main body of that second finger joint module is a second finger joint rotating shaft, the second finger joint rotating shaft is rotatively arranged on a the instrument base housing, the second finger joint rotating shaft can rotate around a central axis of the second finger joint rotating shaft, a fourth twist rope is in power transmission with the second rope guide wheel, one end of the fourth twist rope is wound on the upper part of the second finger joint rotating shaft clockwise, the other end is counterclockwise wound on the lower part of the second finger joint rotating shaft, the second finger joint rotating shaft can drive the second rope guide wheel to rotate in the rotating process, which is used to drive the second jaw to move; the main body of the wrist joint module is a wrist joint rotating shaft which is rotatively arranged on the instrument base housing and can rotate around the central axis thereof, the fifth twist rope is in power transmission with the third rope guide wheel; one end of the fifth twist rope is wound on the upper part of the wrist joint rotating shaft clockwise, and the other end of the fifth twist rope is wound on the lower part of the wrist joint rotating shaft anticlockwise, the wrist joint rotating shaft can drive the third rope guide wheel to rotate in the rotation process, which is used to drive the wrist joint to move.

5. The multi-degree-of-freedom instrument for robot according to claim 4, wherein the instrument base further comprises a mounting buckle which is arranged on the instrument base housing and is used for realizing the clamping connection between the multi-degree-of-freedom instrument for robot and the robot.

6. The multi-degree-of-freedom instrument for robot according to claim 5, wherein the instrument rod is a hollow shaft, and the third twist rope, the fourth twist rope, and the fifth twist rope respectively pass through the instrument rod.

7. The multi-degree-of-freedom instrument for robot according to claim 5, wherein the wrist joint is provided with a pulley block, and the third twist rope and the fourth twist rope respectively bypass the pulley block for reversing and tensioning the third twist rope and the fourth twist rope.

8. The multi-degree-of-freedom instrument for robot according to claim 5, wherein at least three first pulleys are arranged on the instrument base housing, the third twist rope, the fourth twist rope and the fifth twist rope respectively go around the first pulleys corresponding to the third twist rope, the fourth twist rope and the fifth twist rope to realize the direction change of the third twist rope, the forth twist rope and fifth twist rope.
